# EUROPEAN PATENT APPLICATION

(11) **EP 1 764 089 A1**
(43) Date of publication of application: **21.03.2007**
(21) Application number: 05020216.7
(22) Date of filing: 15.09.2005
(51) Int. Cl.: A61K 9/127

(54) **Serum stable liposomes comprising amphoter II lipid mixtures**

(71) Applicant: novosom AG, 06120 Halle (DE)
(72) Inventor: Panzner, Steffen. Dr., 06108 Halle (DE); Lutz, Silke. Dr., 06114 Halle (DE); Rauchhaus, Una. Dr., 06114 Halle (DE); Kerwitz, Yvonne. Dr., 06114 Halle (DE)
(74) Representative: Ziebig, Marlene

(57) **Abstract**

To provide stable formulations of amphoteric liposomes that do not release encapsulated drugs e. g. otigonucteotide drugs upon contact with human serum amphoteric liposomes comprising a mix of lipid components with amphoteric properties and a mixture of phosphatidylcholine and phosphatidylethanolamine whereas the ratio phosphatidylethanolamins to phosphatidylcholins is between 0.5 and 8 are proposed. The serum stable amphoteric liposomal formulations can be used for the intracellular delivery of drugs and for the prevention or treatment of a condition and/or disease in mammals or part of mammals in particular humans or their organs.

## Description

### Field of the invention

Amphoteric liposomes represent a recently described class of liposomes having an anionic or neutral charge at pH7.5 and a cationic charge at pH4. These liposomes are very well tolerated in animals and can encapsulate nucleic acid molecules with high efficiency. The invention provides selected stable formulations of amphoteric liposomes that do not release encapsulated drugs upon contact with human serum. Such liposomal formulations are useful in the delivery of drugs after a systemic application into the blood stream. The invention without being limited towards such use in particular suits the delivery of oligonucleotides, a new class of drugs that is currently under development. Excluding CpG-oligos or aptamers, the majority of these compounds has an intracellular site of action. Carrier systems are used to overcome the poor uptake of such substances and are sometimes an indispensable prerequisite.

### Definitions

Within that disclosure the following definitions should apply:
**Amphoter or amphoteric character:** A structure, being a substance or a mixture of substances or a supramolecular complex (e.g. a liposome) comprising charged groups of both anionic and cationic character wherein
   (i) at least one of the charged groups has a pK between 4 and 8 and
   (ii) the cationic charge prevails at pH 4 and
   (iii) the anionic charge prevails at pH 8,
   resulting in an isoelectric point of neutral net charge between pH4 and pH8. Amphoteric character by that definition is different from zwitterionic character, as zwitterions do not have a pK in the range mentioned above. In consequence, zwitterions are essentially neutrally charged over a range of pH values. Phosphatidylcholine or phosphatidylethanolamins are neutral lipids with zwitterionic character.
**Amphoter I Lipid Pairs:** stable cation and stable chargeable anion.
   Examples include DDAB/CHEMS, DOTAP/CHEMS, DOTAP/DOPS and many more.
**Amphoter II Lipid Pairs:** chargeable cation and chargeable anion.
   Examples include Mo-Chol/CHEMS, DPIM/CHEMS or DPIM/DG-Succ.
**Amphoter III Lipid Pairs:** chargeable cation and stable anion.
   Examples include Mo-Chol/DOPG or Mo-Chol/Chol-SO4.

### Background of the invention

Oligonucleotides represent a novel class of drugs that can very specifically down regulate or interfere with protein expression.

Without being limited to the following, oligonucleotides comprise antisense, locked nucleic acids (LNA), peptide nucleic acids (PNA), morpholino nucleic acids (Morpholinos), small interfering RNAs (siRNA) or transcription factors decoys of various chemistries. A detailed description of the different mechanism can be found in the literature (eg. Crooke in BBA (1999), 1489(1), 31-44; Tijsterman et al. in Cell (2004), 117(1), 1-3 or Mann et al. in J Clin Invest, (2000), 106(9), 1071-5.
Also oligonucleotides for gene repair applications (eg. Richardson et al. in Stem Cells (2002), 20, 105-118), micro RNAs and the like are known examples form that rapidly growing field.

It is known in the art, that nucleic acid therapeutics, irrespective of their actual chemical origin lack therapeutic efficacy due to instability in body fluids or inefficient uptake into cells or for both reasons. Chemical modifications of the oligonucleotide comprising but not limited to the abovementioned variants, also extended towards the formation of conjugates with ligands or polymers represent one strategy to overcome practical limitations. A second set of strategies involves the use of carrier systems, in particular liposomes for protection, targeting and enhanced uptake into cells. Such carrier system shall meet an optimum score of the following criteria: high encapsulation efficiency and economic production process, colloidal stability of the carrier, enhanced uptake into cells and of course low toxicity and immunogenicity.

Anionic or neutral liposomes are often excellent in terms of colloidal stability, as no aggregation occurs between the carrier and the environment. Consequently their biodistribution is excellent and the potential for irritation and cytotoxicity is low. However, such carrier lack encapsulation efficiency and do not provide an endosomolytic signal that facilitates further uptake into cells (Journal of Pharmacology and experimental Therapeutics (2000), 292, 480-488 by Klimuk et al.).

A great many of publications deal with cationic liposomal systems, e.g. Molecular Membrane Biology (1999), 16, 129-140 by Maurer et al. ; BBA (2000) 1464, 251-261 by Meidan et al.; Reviews in Biology and Biotechnology (2001), 1(2), 27-33 by Fiset & Gounni . Although cationic systems provide high loading efficiencies, they lack colloidal stability, in particular after contact with body fluids. Ionic interactions with proteins and/or other biopolymers lead to in situ aggregate formation with the extracellular matrix or with cell surfaces. Cationic lipids have often been found to be toxic as shown by Filion et al. in BBA (1997), 1329(2), 345-356; Dass in J. Pharm. Pharmacol. 2002), 54(5), 593-601; Hirko et al. in Curr. Med. Chem., 10(14), 1185-1193.

These limitations were overcome by the addition of components that provide a steric stabilization to the carriers. Polyethylenglycols of various chain length are known to eliminate the aggregation problems associated with the use of cationic components in body fluids and PEGylated cationic liposomes show enhanced circulation times in vivo (BBA (2001) 1510, 152-166 by Semple et al.). Still, the use of PEG does not solve the intrinsic toxicity problem associated with the cationic lipids. It is also known that PEG substantially inhibits the productive entry of such liposomes into the cells or their intracellular delivery (Song et al. in BBA (2002), 1558(1), 1-13. Quite recently, Morrissey et al. (Nature Biotechnology (2005), 23 (8), 1002 - 1007) described a diffusible PEG-lipid for a cationic vector that is able to transfer siRNA into liver cells in vivo. Still, the huge demand for such solutions the development and the given attrition rate of clinical development more than motivates the development in particular of conceptually independent solution.

Amphoteric liposomes represent a recently described class of liposomes having an anionic or neutral charge at pH7.5 and a cationic charge at pH4. Explicit reference is made here to WO 02/066490, WO 02/066012 and the WO 03/070735, all to Panzner et al. which give a detailed description of the amphoteric liposomes and suitable lipids therefore. Further disclosures are made in WO 03/070220 and WO 03 070735, also to Panzner et al. describing more pH sensitive lipids for the manufacturing of said amphoteric liposomes. Amphoteric liposomes have an excellent biodistribution and are very well tolerated in animals. They can encapsulate nucleic acid molecules with high efficiency.

The use of amphoteric liposomes as carriers for drugs for the prevention or treatment of different conditions or diseases in mammals requires stability of the liposomes after the injection of the liposomes into the bloodstream. Especially after a systemic application the drug must be stable encapsulated in the liposomes until eventual uptake in the target tissue or cells. Therefore the guidelines for liposomal formulations of the FDA regulate specific preclinical tests for liposomal drugs (http://www.fda.gov/cder/guidance/2191dft.pdf). For example, the ratio of encapsulated drug to free drug must be determined during the circulation time in the blood stream.
After the injection of liposomes into the blood stream serum components are interact with the liposomes and lead to a permeabilization of the liposomal membrane. However, the release of a drug that is encapsulated in a liposome depends on the molecular dimensions of the drug. This means that a plasmid drug with a size of thousands of base pairs is released much more slowly than smaller oligonucleotides or other small molecules. For a liposomal delivery of drugs it is essential that the release of the drug during the circulation of the liposomes is as low as possible.

### Object of the invention

Therefore, the object of the invention described here is to provide stable formulations of amphoteric liposomes that do not release encapsulated drugs upon contact with human serum.

Another object of the invention is therefore to provide delivery systems for oligonucleotide drugs.

### Summary of the invention

The present invention is directed to amphoteric liposomes comprising a mix of lipid components with amphoteric properties and neutral lipids. The amphoteric liposomes are negatively or neutrally charged at pH 7.4 and cationic at pH 4. The invention relates to preferred types amphoteric liposomes. More specifically, the invention relates to amphoteric liposomes having both a pH sensitive anionic and a pH sensitive cationic lipid. In a preferred embodiment of the invention oligonucleotide drugs are encapsulated into said amphoteric liposomes. A substantial portion or all of said oligonuclotides is physically entrapped into the amphoteric liposomes. The serum stable amphoteric liposomal formulations can be used for the intracellular delivery of drugs and for the prevention or treatment of a condition and/or disease in mammals or part of mammals in particular humans or their organs.

### Detailed description of the invention

Amphoteric liposomes of the present invention comprise a mix of lipid components with amphoteric properties and a mixture of phosphatidylcholine and phosphatidylethanolamine.

The ratio phosphatidylethanolamins to phosphatidylcholins is preferred between 0.5 and 8, more preferred between 0.75 and 5 and most preferred between 1 and 4.
Preferred components are selected from the group of DMPC, DPPC, DSPC, POPC, DOPC or from phosphatidylcholins from natural sources such as soy bean PC or egg PC. Preferred phosphatidylethanolamins are selected from DOPE or DMPE or DPPE.
Most preferred neutral lipids are DOPE, POPC, soy bean PC or egg PC.

Also, it is known that cholesterol stabilizes phosphatidylcholine bilayers against serum attack. Although neither POPC nor DOPE form serum stable structures by themselves, it surprisingly was found that mixtures of DOPE and POPC form serum stable liposomes. Such structures are clearly do not fall under the definition of amphoters given above, but might be useful as a carrier as well.
The ratio phosphatidylethanolamins to phosphatidylcholins in such mixtures lacking the amphoteric lipid pair is preferred between 0.5 and 8, more preferred between 0.75 and 5 and most preferred between 1 and 4.
Preferred components are selected from the group of DMPC, DPPC, DSPC, POPC, DOPC or from phosphatidylcholins from natural sources such as soy bean PC or egg PC. Preferred phosphatidylethanolamins are selected from DOPE or DMPE or DPPE.
Most preferred neutral lipids are DOPE, POPC, soy bean PC or egg PC.

Amphoteric liposomes of the present invention comprise anionic and cationic components, wherein both components are pH-sensitive as disclosed in WO 02/066012. Cationic lipids sensitive to pH are disclosed in WO 02/066489 A2/A3 and WO 03/070220 A1 and in the references made therein, in particular in Budker et al. 1996, Nat Biotechnol. 14(6):760-4.
Preferred cationic components are selected from the group of MoChol, HisChol, DPIM, CHIM. More preferred, the cationic component is MoChol or HisChol with MoChol being the most preferred component.
The amphoteric mixtures further comprise anionic lipids and preferred lipids for use with the invention are selected from the group comprising: DOGSucc, POGSucc, DMGSucc, DPGSucc and CHEMS.
Further preferred anionic lipids are DOGSucc, DMGSucc and CHEMS.

Abbreviations for lipids refer primarily to standard use in the literature and are included here as a helpful reference:
- PC: Phosphatidylcholine, unspecified membrane anchor
- PE: Phosphatidylethanolamine, unspecified membrane anchor
- DMPC: Dimyristoylphosphatidylcholin
- DPPC: Dipalmitoylphosphatidylcholin
- DSPC: Distearoylphosphatidylcholin
- POPC: Palmitoyl-oleoylphosphatidylcholin
- DOPC: Dioleoylphosphatidylcholin
- Chems: Cholesterolhemisuccinat
- DPIM: 4-(2,3-bis-palmitoyloxy-propyl)-1-methyl-1H-imidazole
- CHIM: Cholesterol-(3-imidazol-1-yl propyl)carbamate
- MoChol: 4-(2-Aminoethyl)-Morpholino-Cholesterolhemisuccinat
- HisChol: Histaminyl-Cholesterolhemisuccinat.
- DGSucc: 1,2-Dipalmitoyglycerol-3-hemisuccinat & Distearoyl-, dimyristoyl- Dioleoyl or palmitoyl-oleoylderivatives

| | |
|---|---|
| 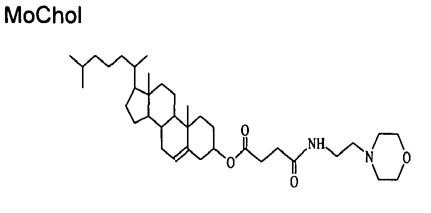 | 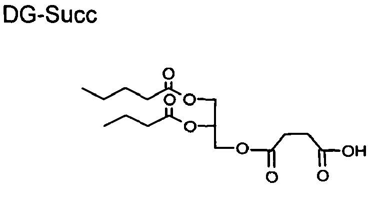 |
| 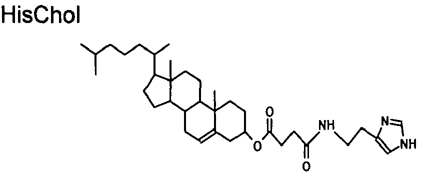 | |

It was found, that the ratio between the cationic and the anionic lipid (the charge ratio) not only determines the isoelectric point but also affects the serum stability of the composition. The charge ratio might vary from 4:1 to 1:4, more preferred between 3:1 and 1:3 (cation:anion).

In a preferred embodiment of the invention the cation is present in excess over the anion. More preferred such charge ratio is between 3:1 and 2:1. The total amount of charged lipids may vary from 5 to 95% of the lipid mixture, more preferred from 30 to 80% and even more preferred from 50 to 75% with the remaining lipids being formed from the neutral phospholipids PC and PE.

In another preferred embodiment of the invention cation and anion are present in equal amounts. The total amount of charged lipids may vary from 5 to 75% of the lipid mixture, more preferred from 20 to 65% and even more preferred from 30 to 50% with the remaining lipids being formed from the neutral phospholipids PC and PE.

In yet another preferred embodiment of the invention the anion is present in excess over the cation. More preferred, such charge ratio is between 1:3 and 1:2, even more preferred the charge ratio is about 1:2 (cation:anion). The total amount of charged lipids may vary from 40 to 75% of the lipid mixture, more preferred from 50 to 70% and even more preferred from 55 to 65% with the remaining lipids being formed from the neutral phospholipids PC and PE.

A number of combinations of different cations and anions can be selected from the list of suitable components given above. With preference, the invention is practiced using MoChol as cation and CHEMS or DMG-Succ as the anion.
Even more preferred, liposomes are made from a mixture of lipids comprising POPC and DOPE in a ratio between 1:1 and 1:4 and further comprising MoChol and CHEMS in a ratio between 3:1 and 1:1, wherein the amount of charged lipids is between 30 and 80% of the lipid mixture.

Very specific examples of such liposomes include, but are not limited to:
- POPC/DOPE/MoChol/Chems: 6:24:53:17
- POPC/DOPE/MoChol/Chems: 6:24:47:23
- POPC/DOPE/MoChol/Chems: 15:45:20:20
- POPC/DOPE/MoChol/Chems: 10:30:30:30
- POPC/DOPE/MoChol/Chems: 24.5:35.5:20:20
- POPC/DOPE/MoChol/Chems: 16:24:30:30

Also, liposomes are made from a mixture of lipids comprising POPC and DOPE in a ratio between 1:1 and 1:4 and further comprising MoChol and DMG-Succ in a ratio between 3:1 and 1:3, wherein the amount of charged lipids is between 30 and 80%. Even more preferred, the charge ratio is between 1:2 and 1:3 and charged components consitute between 50 and 70% of the lipid mixture.
Very specific examples of such liposomes include, but are not limited to:
- POPC/DOPE/MoChol/DMG-Succ: 6:24:53:17
- POPC/DOPE/MoChol/DMG-Succ: 6:24:47:23
- POPC/DOPE/MoChol/DMG-Succ: 6:24:23:47
- POPC/DOPE/MoChol/DMG-Succ: 8:32:20:40
- POPC/DOPE/MoChol/DMG-Succ: 10:40:17:33
- POPC/DOPE/MoChol/DMG-Succ: 10:20:23:47
- POPC/DOPE/MoChol/DMG-Succ: 13:27:20:40
- POPC/DOPE/MoChol/DMG-Succ: 17:33:17:33
- POPC/DOPE/MoChol/DMG-Succ: 15:45:20:20
- POPC/DOPE/MoChol/DMG-Succ: 10:30:30:30
- POPC/DOPE/MoChol/DMG-Succ: 24.5:35.5:20:20
- POPC/DOPE/MoChol/DMG-Succ: 16:24:30:30

Without being limited towards such use, the materials described in the present invention are in particular suited as carriers for oligonucleotides. Such oligonucleotides as active drugs can target or outcompete existing intracellular nucleic acids. The term "target nucleic acid" encompass DNA encoding for a specific protein as well as all RNAs derived from such DNA, being pre-mRNA or mRNA. A specific hybridisation between the target nucleic acid and one or more oligonucleotides directed against such sequences result in an inhibition of protein expression. To achieve such specific targeting, the oligonucleotide must comprise a continuous stretch of nucleotides being complementary to the sequence of the target nucleic acid.

Oligonucleotides fulfilling the abovementioned criteria may be built with a number of different chemistries and topologies. Oligonucleotides may be single stranded or double stranded. Single stranded oligonucleotides include but are not limited to DNA-based oligonucleotides, locked nucleic acids, 2'-modified oligonucleotides and others, commonly known as antisense oligonucleotides. Backbone or base modifications may include but are not limited to Phosphothioate DNA (PTO), 2'O-methyl RNA (2'Ome), 2' O- methoxyethyl-RNA (2'MOE), peptide nucleic acids (PNA), N3'-P5' phosphoamidates (NP), 2'fluoroarabino nucleic acids (FANA), locked nucleic acids (LNA), Morpholine phosphoamidate (Morpholino), Cyclohexene nucleic acid (CeNA), tricyclo-DNA (tcDNA) and others. Moreover, mixed chemistries are known in the art, being constructed from more than a single nucleotide species as copolymers, block-copolymers or gapmers or in other arrangements.

In addition to the aforementioned oligonucleotides, a protein expression can also be inhibited using double stranded RNA molecules containing the complementary sequence motifs. Such RNA molecules are known as siRNA molecules in the art (eg. WO9932619A1 or WO02055693A2). Again, various chemistries were adapted to this class of oligonucleotides. Also, DNA / RNA hybrid systems are known in the art.
In another embodiment of the present invention, decoy oligonucleotides can be used. These double stranded DNA molecules do not target nucleic acids but transcription factors. This means that decoy oligonucleotides bind sequence-specific DNA-binding proteins and interfere with the transcription (eg. Cho-Chung et al. in Curr Opin Mol Ther., 1999).

All above mentioned oligonucleotides may vary in length between as little as 10, preferably 15 and even more preferably 18 and 50, preferably 30 and more preferably 25 nucleotides. The fit between the oligonucleotide and the target sequence is preferably perfect with each base of the oligonucleotide forming a base pair with its complementary base on the target nucleic acid over a continuous stretch of the abovementioned number of oligonucleotides. The pair of sequences may contain one mismatch within the said continuous stretch of base pairs, although this is less preferred.

Methods for the manufacturing of lipoosmes are known to those skilled in the art. They include but are not limited to extrusion through membranes of defined pore size, injection of lipid solutions in ethanol into the water phase containing cargo or high pressure homogenization.
Also, it is known in the art that nucleic acid therapeutics can be contacted with the lipids at neutral pH, resulting in volume inclusion of a certain percentage of the solution containing the nucleic acid. High concentrations of lipids ranging from 50mM to 150mM are preferred to achieve substantial encapsulation of the drug.

In contrast to such standard procedure, amphoteric liposomes offer the distinct advantage of binding nucleic acids at or below their isoelectric point and thereby concentrating the drug at the liposome surface. Such process is described in WO 02/066012 A2/A3 in more detail.

Irrespective of the actual production process the non-encapsulated active drug can be removed from the liposomes after the initial production step wherein liposomes are formed as tight containers. Again, the technical literature and the references included here describe such methodology in detail and suitable process steps may include but are not limited to size exclusion chromatography, sedimentation, dialysis, ultrafiltration or diafiltration and the like.
In a preferred embodiment of the invention more than 80% of the drug are inside the liposomes.
However, such removal of non-encapsulated material is not mandatory and in one preferred embodiment of the invention the formulation comprises entrapped as well as free drug.

The particle size of the formulation is between 50 and 500nm, more preferred between 50 and 300nm and even more preferred between 50 and 200nm.
While the present invention has been described with specificity in accordance with certain of its preferred embodiments, the following examples serve only to illustrate the invention and are not intended to limit the same.

### Example 1: Preparation of carboxyfluorescein (CF) loaded liposomes with the amphoteric II lipids MoChol and Chems

Stock solutions of lipids in chloroform were mixed and finally evaporated in a round bottom flask to dryness under vacuum. Lipid films were hydrated with 100 mM CF in PBS pH 7.5. The resulting lipid concentration was 20 mM. The suspensions were hydrated for 45 minutes in a water bath at room temperature, sonicated for 5 minutes following by three freeze/thaw cycles at -70°C. After thawing the liposomal suspensions were extruded 15 times through polycarbonate membranes with a pore size of 100nm. Non-encapsulated CF was removed by gel filtration, whereas the liposomes were diluted by a factor three.

**Tab.1: Variation of the ratio DOPE/POPC and the total amount of charged components**

| **Lipids** | **Composition** |
|---|---|
| DOPE/MoChol/Chems | 60:20:20 |
| DOPE/MoChol/Chems | 50:20:30 |
| DOPE/MoChol/Chems | 40:30:30 |
| DOPE/MoChol/Chems | 20:40:40 |
| POPC/MoChol/Chems | 60:20:20 |
| POPC/MoChol/Chems | 40:30:30 |
| POPC/MoChol/Chems | 20:40:40 |
| POPC | 100 |
| POPC/DOPE | 20:80 |
| POPC/DOPE/MoChol/Chems | 10:50:20:20 |
| POPC/DOPE/MoChol/Chems | 7:35:30:30 |
| POPC/DOPE/MoChol/Chems | 3:17:40:40 |
| POPC/DOPE | 25:75 |
| POPC/DOPE/MoChol/Chems | 15:45:20:20 |
| POPC/DOPE/MoChol/Chems | 10:30:30:30 |
| POPC/DOPE/MoChol/Chems | 5:15:40:40 |
| POPC/DOPE | 40:60 |
| POPC/DOPE/MoChol/Chems | 24.5:35.5:20:20 |
| POPC/DOPE/MoChol/Chems | 16:24:30:30 |
| POPC/DOPE/MoChol/Chems | 8:12:40:40 |
| POPC/DOPE | 57:43 |
| POPC/DOPE/MoChol/Chems | 34:26:20:20 |
| POPC/DOPE/MoChol/Chems | 22.8:17.2:30:30 |
| POPC/DOPE/MoChol/Chems | 11.4:8.6:40:40 |

**Tab.2: Variation of the ratio MoChol/Chems and the total amount of charged components**

| **Lipids** | **Composition** |
|---|---|
| POPC/DOPE/MoChol/Chems | 6:24:53:17 |
| POPC/DOPE/MoChol/Chems | 6:24:47:23 |
| POPC/DOPE/MoChol/Chems | 6:24:35:35 |
| POPC/DOPE/MoChol/Chems | 6:24:23:47 |

**Tab.3: Variation of ratio DOPE/POPC and the total amount of charged components**

| **Lipids** | **Composition** |
|---|---|
| POPC/DOPE/MoChol/Chems | 4:16:26:53 |
| POPC/DOPE/MoChol/Chems | 6:24:23:46 |
| POPC/DOPE/MoChol/Chems | 8:32:20:40 |
| POPC/DOPE/MoChol/Chems | 10:40:17:33 |
| POPC/DOPE/MoChol/Chems | 7:13:26:53 |
| POPC/DOPE/MoChol/Chems | 10:20:23:46 |
| POPC/DOPE/MoChol/Chems | 13:26:20:40 |
| POPC/DOPE/MoChol/Chems | 17:33:17:33 |

### Example 2: Preparation of carboxyfluorescein (CF) loaded liposomes with the amphoteric II lipids MoChol and DMG-Succ

Liposomes were prepared as described in example 1.

**Tab.4: Variation of the ratio DOPE/POPC and the total amount of charged components**

| **Lipids** | **Composition** |
|---|---|
| POPC/DOPE/MoChol/DMG-Succ | 15:45:20:20 |
| POPC/DOPE/MoChol/DMG-Succ | 10:30:30:30 |
| POPC/DOPE/MoChol/DMG-Succ | 5:15:40:40 |
| POPC/DOPE/MoChol/DMG-Succ | 24.5:35.5:20:20 |
| POPC/DOPE/MoChol/DMG-Succ | 16:24:30:30 |
| POPC/DOPE/MoChol/DMG-Succ | 8:12:40:40 |
| POPC/DOPE/MoChol/DMG-Succ | 34:26:20:20 |
| POPC/DOPE/MoChol/DMG-Succ | 22.8:17.2:30:30 |
| POPC/DOPE/MoChol/DMG-Succ | 11.4:8.6:40:40 |

**Tab.5: Variation of the ratio MoChol/DMG-Succ and the total amount of charged components**

| **Lipids** | **Composition** |
|---|---|
| POPC/DOPE/MoChol/DMG-Succ | 6:24:53:17 |
| POPC/DOPE/MoChol/DMG-Succ | 6:24:47:23 |
| POPC/DOPE/MoChol/DMG-Succ | 6:24:35:35 |
| POPC/DOPE/MoChol/DMG-Succ | 6:24:23:47 |

**Tab.6: Variation of ratio DOPE/POPC and the total amount of charged components**

| **Lipids** | **Composition** |
|---|---|
| POPC/DOPE/MoChol/DMG-Succ | 4:16:26:53 |
| POPC/DOPE/MoChol/DMG-Succ | 6:24:23:46 |
| POPC/DOPE/MoChol/DMG-Succ | 8:32:20:40 |
| POPC/DOPE/MoChol/DMG-Succ | 10:40:17:33 |
| POPC/DOPE/MoChol/DMG-Succ | 7:13:26:53 |
| POPC/DOPE/MoChol/DMG-Succ | 10:20:23:46 |
| POPC/DOPE/MoChol/DMG-Succ | 13:26:20:40 |
| POPC/DOPE/MoChol/DMG-Succ | 17:33:17:33 |

### Example 3: Serum stability test of CF-loaded amphoteric liposomes of examples 1 and 2

Carboxyfluorescein (CF) was used as model drug to determine the serum stability of amphoteric liposomes. As well as oligonucleotides CF is negatively charged. The serum stability was observed over a period of 4 hours by determining the release of CF from the liposomes via fluorescence measurement. The released amount of CF (in %) is measured at defined time points as well as after a treatment of the liposomes with a detergent (Triton X-100) to get a 100 % release value.
Liposomes were incubated in full human serum at 37 °C. The final lipid concentration was 5 mM. At defined time points 5 µl sample was transferred into a 96-well microtiter plate to 20 µl PBS, pH 7.5 or 20 µl 20% Triton X-100. Finally 275 µl PBS were added to each well and fluorescence intensity was measured at 475/530 nm.

### Results:

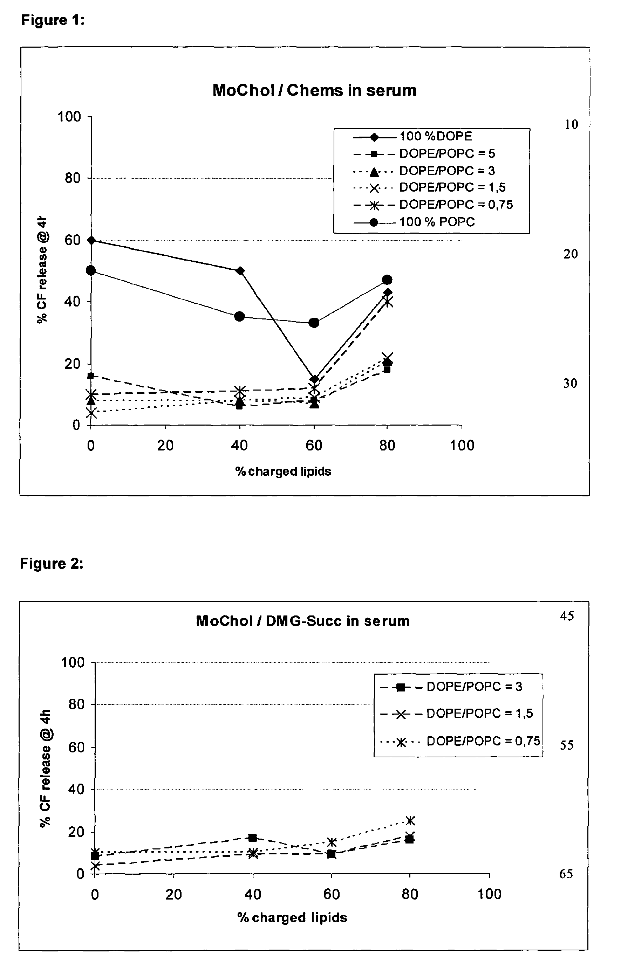

Mixtures of POPC and DOPE are stable in serum. POPC itself does not form liposomes that withstand attack from serum. In addition, DOPE does not form liposomes at all. Quite surprisingly, mixtures from both components were found to be very stable and resistant against serum attack. POPC/DOPE ratios from 0,75 to 5 were found to form stable structures with a broad optimum between 1,5 and 5 (see also figure 1 and 2).

Charged components and neutral lipids are independent variables. Serum sensitivity for a 1:1 ratio of both MoChol/CHEMS or MoChol/DMG-Succ is low to very low and stable particles are formed over a wide range of mixtures. It needs at least 60 or 70% of total charged components to significantly affect the bilayer stability.

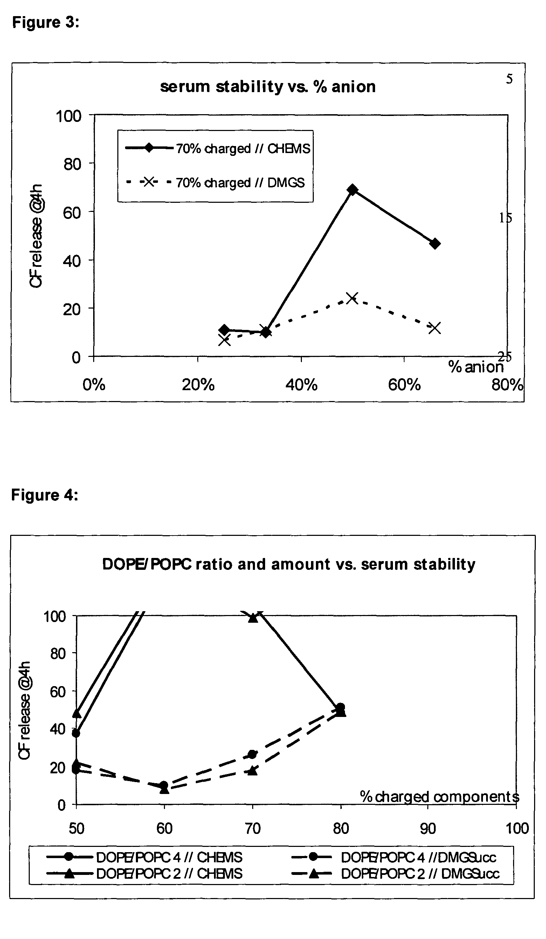

The serum stability of lipid mixtures containing 70% of charged components (see table 2 and 5) is shown in figure 3. In general, an excess of MoChol has a stabilizing effect.

The formulations of table 3 and 6 that were tested for serum stability have DOPE and POPC in a ratio of either 2:1 or 4:1. The total amount of the charged lipids was titrated from 80 % down to 50%. The results are shown in figure 4.

Figure legends
- **Figure 1:**: Serum release from MoChol/Chems formulations of table 1 after incubation in full human serum for 4 hours. Release is expressed as % of the unquenched CF signal. Y-axis shows total amount of charged lipid at a 1:1 ratio between MoChol and Chems.
- **Figure 2:**: Serum release from MoChol/DMG-Succ formulations of table 4 after incubation in full human serum for 4 hours. Release is expressed as % of the unquenched CF signal. Y-axis shows total amount of charged lipid at a 1:1 ratio between MoChol and DMG-Succ.
- **Figure 3:**: Serum release from MoChol/Chems or MoChol/DMG-Succ after incubation in full human serum at 37°C. Release is expressed as % of the unquenched CF signal. Excess of the cation stabilizes the liposomes against serum attack.
DMG-Succ is notably more stable then the CHEMS counterpart.
- **Figure 4:**: Serum release from MoChol/CHEMS- and MoChol/DMG-Succ-formulations of tables 3 and 6. Formulations have DOPE/POPC ratios of 2 and 4 and the ratio cationic to anionic lipid is less than 1 (see also table 3 and 6) after incubation in full human serum at 37°C. Release is expressed as % of the unquenched CF signal.

## Claims

1. Amphoteric liposomes comprising a mix of lipid components with amphoteric properties and a mixture of phosphatidylcholine and phosphatidylethanolamine whereas the ratio phosphatidylethanolamins to phosphatidylcholins is preferred between 0.5 and 8, more preferred between 0.75 and 5 and most preferred between 1 and 4.

2. Amphoteric liposomes of claim 1 wherein the ratio between the cationic and the anionic lipid (the charge ratio) might vary from 4:1 to 1:4, more preferred between 3:1 and 1:3 (cation:anion).

3. Amphoteric liposomes of claim 1 wherein the cation is present in excess over the anion and the charge ratio cation:anion is between 3:1 and 2:1 and the total amount of charged lipids may vary from 5 to 95% of the lipid mixture, more preferred from 30 to 80% and even more preferred from 50 to 75% with the remaining lipids being formed from the neutral phospholipids PC and PE.

4. Amphoteric liposomes of claim 1 wherein the cation and anion are present in equal amounts and the total amount of charged lipids may vary from 5 to 75% of the lipid mixture, more preferred from 20 to 65% and even more preferred from 30 to 50% with the remaining lipids being formed from the neutral phospholipids PC and PE.

5. Amphoteric liposomes of claim 1 wherein the anion is present in excess over the cation and the charge ratio cation:anion is between 1:3 and 1:2, even more preferred the charge ratio is about 1:2 (cation:anion) and the total amount of charged lipids may vary from 40 to 75% of the lipid mixture, more preferred from 50 to 70% and even more preferred from 55 to 65% with the remaining lipids being formed from the neutral phospholipids PC and PE.

6. Amphoteric liposomes of claim 1 wherein the liposomes are made from a mixture of lipids comprising POPC and DOPE in a ratio between 1:1 and 1:4 and further comprising MoChol and CHEMS in a ratio between 3:1 and 1:1, wherein the amount of charged lipids is between 30 and 80% of the lipid mixture.

7. Amphoteric liposomes of claim 1 wherein the liposomes are made from a mixture of lipids comprising POPC and DOPE in a ratio between 1:1 and 1:4 and further comprising MoChol and DMG-Succ in a ratio between 3:1 and 1:3, wherein the amount of charged lipids is between 30 and 80%. Even more preferred, the charge ratio is between 1:2 and 1:3 and charged components constitute between 50 and 70% of the lipid mixture.

8. Amphoteric liposomes of claim 1 wherein the phosphatidylcholines are selected from the group of DMPC, DPPC, DSPC, POPC, DOPC or from phosphatidylcholins from natural sources such as soy bean PC or egg PC and the phosphatidylethanolamins are selected from DOPE or DMPE or DPPE.

9. Amphoteric liposomes of claim 8 wherein the phosphatidylethanolamins and phosphatidylcholines are selected from DOPE, POPC, soy bean PC or egg PC.

10. Amphoteric liposomes of claim 1 wherein the amphoteric lipid is a mixture comprising two or more different anionic and cationic lipids selected from the group of MoChol, HisChol, DPIM, CHIM, DOGSucc, POGSucc, DMGSucc, DPGSucc and Chems.

11. Amphoteric liposomes of claim 10 wherein the amphoteric lipid mixtures comprise one or more from the group of MoChol, HisChol, DOGSucc, DMGSucc and Chems.

12. Amphoteric liposomes of claim 1 having a size between 50 and 500nm and, more preferentially having a size between 50 and 200nm.

13. Amphoteric liposomes of one or more of the claims 1 to 12 wherein the liposomes comprise one or more drugs in the aqueous core.

14. Amphoteric liposomes of claim 13 wherein the drug is a oligonucleotide.

15. Amphoteric liposomes of claim 14 wherein the oligonucleotide is an antisense oligonucleotide of 15 to 30 basepairs length.

16. Amphoteric liposomes of claim 14 wherein the oligonucleotide contains phosphothioate linkages.

17. Amphoteric liposomes of claim 14 wherein the oligonucleotide contains 2'MOE modified nucleobases.

18. Amphoteric liposomes of claim 14 wherein the oligonucleotide is a siRNA of 15 to 30 basepairs length.

19. Amphoteric liposomes of claim 14 wherein the oligonucleotide is a decoy oligonucleotide of 15 to 30 basepairs length.

20. Amphoteric liposomes of one or more of the claims 1 to 19 wherein more than 80 % of the oligonucleotide are inside the liposomes.

21. Amphoteric liposomes of one or more of the claims 1 to 20 wherein non-encapsulated oligonucleotides are not removed from the liposomal suspension.

22. Amphoteric liposomes of one or more of the claims 1 to 21 wherein the molar composition is POPC/DOPE/MoChol/Chems 15:45:20:20.

23. Amphoteric liposomes of one or more of the claims 1 to 21 wherein the molar composition is POPC/DOPE/MoChol/Chems 10:30:30:30.

24. Amphoteric liposomes of one or more of the claims 1 to 21 wherein the molar composition is POPC/DOPE/MoChol/DMG-Succ 10:30:20:40.

25. Amphoteric liposomes of one or more of the claims 1 to 21 wherein the molar composition is POPC/DOPE/MoChol/DMG-Succ 6:24:47:23.

26. Pharmaceutical composition comprising amphoteric liposomes of one or more of the claims 1 to 25.

27. Use of the amphoteric liposomes of one or more of the claims 1 to 25 for the production of pharmaceutical compositions for the prevention or treatment of a condition and/or disease in mammals or part of mammals in particular humans or their organs.
